(19) Europäisches Patentamt
European Patent Office
Office européen des brevets

(11) EP 3 618 625 B1

(12) EUROPEAN PATENT SPECIFICATION

(45) Date of publication and mention of the grant of the patent:
**02.03.2022 Bulletin 2022/09**

(21) Application number: **18794937.5**

(22) Date of filing: **02.05.2018**

(51) International Patent Classification (IPC):
***A01N 43/54*** (2006.01) ***A01N 43/653*** (2006.01)
***C07C 229/08*** (2006.01) ***A01P 3/00*** (2006.01)
***A01N 43/40*** (2006.01)

(52) Cooperative Patent Classification (CPC):
(C-Sets available)
**A01N 43/40** (Cont.)

(86) International application number:
**PCT/US2018/030560**

(87) International publication number:
**WO 2018/204437 (08.11.2018 Gazette 2018/45)**

(54) **SYNERGISTIC MIXTURES FOR FUNGAL CONTROL IN VEGETABLES**

SYNERGISTISCHE MISCHUNGEN ZUR BEKÄMPFUNG VON PILZBEFALL BEI GEMÜSE

MÉLANGES SYNERGIQUES POUR LA LUTTE CONTRE DES CHAMPIGNONS DANS DES LÉGUMES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.05.2017 US 201762500186 P**

(43) Date of publication of application:
**11.03.2020 Bulletin 2020/11**

(73) Proprietor: **Corteva Agriscience LLC**
**Indianapolis, IN 46268 (US)**

(72) Inventors:
- **YAO, Chenglin**
**Indianapolis, IN 46268 (US)**
- **MATHIESON, John, T.**
**Indianapolis, IN 46268 (US)**

(74) Representative: **f & e patent**
**Braunsberger Feld 29**
**51429 Bergisch Gladbach (DE)**

(56) References cited:
**EP-A2- 0 002 940**
**WO-A1-2016/109257**
**WO-A1-2016/122802**
**TW-B- 150 127**
**US-A- 5 476 868**
**US-A1- 2011 082 160**
**US-A1- 2014 187 587**
**US-A1- 2015 181 874**
**US-B2- 8 883 811**

- **ANONYMOUS ED - DARL KUHN: "Synergistic Fungicidal Compositions of Heterocyclic Aromatic Amides and Triazoles", IP.COM, IP.COM INC., WEST HENRIETTA, NY, US, 20 July 2004 (2004-07-20), XP013020905, ISSN: 1533-0001**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A01N 43/40, A01N 37/34, A01N 43/54,
A01N 43/56, A01N 43/653, A01N 47/14,
A01N 47/24**

**Description**

CROSS REFERENCE TO RELATED APPLICATIONS

This application claims the benefit of U.S. Provisional Patent Application Serial No. 62/500186 filed May 2, 2017.

FIELD

This disclosure concerns the use of a synergistic fungicidal composition containing (a) the compound of Formula I and (b) at least one fungicide selected from the group consisting of a sterol biosynthesis inhibitor selected from the group consisting of prothioconazole, epoxiconazole, and difenoconazole; a strobilurin selected from the group consisting of pyraclostrobin, azoxystrobin, and picoxystrobin; a succinate dehydrogenase inhibitor, for example fluxapyroxad, benzovindiflupyr, penthiopyrad, and bixafen; and a multi-site inhibitor selected from the group consisting of mancozeb and chlorothalonil to provide fungal control in vegetables, as well as a method for the control or prevention of fungal attack in vegetables according to the attached claims.

BACKGROUND AND SUMMARY

Fungicides are compounds, of natural or synthetic origin, which act to protect plants against damage caused by fungi. Current methods of agriculture rely heavily on the use of fungicides. In fact, some crops cannot be grown usefully without the use of fungicides. Using fungicides allows a grower to increase the yield and the quality of the crop, and consequently, increase the value of the crop. In most situations, the increase in value of the crop is worth at least three times the cost of the use of the fungicide.

However, no one fungicide is useful in all situations and repeated usage of a single fungicide frequently leads to the development of resistance to that and related fungicides. Consequently, research is being conducted to produce fungicides and combinations of fungicides that are safer, that have better performance, that require lower dosages, that are easier to use, and that cost less.

Synergism occurs when the activity of two or more compounds exceeds the activities of the compounds when used alone.

It is an object of this disclosure to provide processes using synergistic compositions comprising fungicidal compounds. The synergistic compositions are capable of preventing or curing, or both, diseases caused by fungi of the classes *Ascomycetes* and *Basidiomycetes.* In addition, the synergistic compositions have improved efficacy against the *Ascomycete* and *Basidiomycete* pathogens, including tomato early blight. In accordance with this disclosure, methods for using the synergistic compositions are provided.

DETAILED DESCRIPTION

The present disclosure concerns the use of a synergistic fungicidal mixture comprising a fungicidally effective amount of (a) the compound of Formula I and (b) at least one fungicide selected from the compounds of the following groups A.1, B.1 and C.1:

A.1 Sterol biosynthesis inhibitors (SBI fungicides) selected from the following group a):

a) C14 demethylase inhibitors (DMI fungicides) selected from the group consisting of prothioconazole, epoxiconazole, and difenoconazole;

B.1 Respiration inhibitors selected from the following groups a) and b):

a) inhibitors of complex II (SDHI fungicides, e.g. carboxamides) selected from the group consisting of fluxapyroxad, benzovindiflupyr, penthiopyrad, and bixafen;
b) inhibitors of complex III at the $Q_o$ site (e.g. strobilurins), selected from the group consisting of pyraclostrobin, azoxystrobin, and picoxystrobin;

C.1 Inhibitors with multi-site action selected from the following groups a) and b):

a) thio- and dithiocarbamates selected from the group consisting of mancozeb;
b) organochlorine compounds (e.g. phthalimides, sulfamides, chloronitriles) selected from the group consisting of chlorothalonil;

to provide control of any plant fungal pathogen.

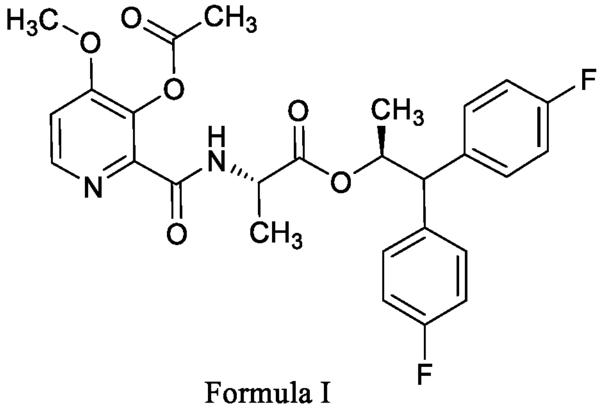

Formula I

As used herein, the compound of Formula I is (*S*)-1,1-bis(4-fluorophenyl)propan-2-yl (3-acetoxy-4-methoxypicolinoyl)-L-alaninate. The compound of Formula I provides control of a variety of pathogens in economically important crops including, but not limited to, the causal agent of tomato early blight, *Alternaria solani* (ALTESO).

As used herein, epoxiconazole is the common name for (2*RS*,3*SR*)-1-[3-(2-chlorophenyl)-2,3-epoxy-2-(4-fluorophenyl)propyl]-1*H*-1,2,4-triazole and possesses the following structure:

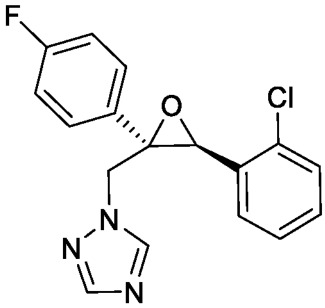

Its fungicidal activity is described in *The Pesticide Manual,* Fifteenth Edition, 2009. Epoxiconazole provides broad spectrum control, with preventive and curative action, of diseases caused by Ascomycetes, Basidiomycetes and Deuteromycetes in bananas, cereals, coffee, rice and sugar beet.

As used herein, prothioconazole is the common name for 2-[(2*RS*)-2-(1-chlorocyclopropyl)-3-(2-chlorophenyl)-2-hydroxypropyl]-2*H*-1,2,4-triazole-3(4*H*)-thione and possesses the following structure:

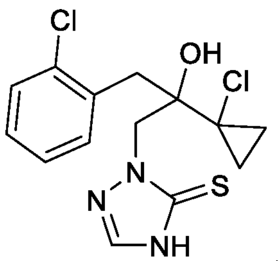

Its fungicidal activity is described in The Pesticide Manual, Fifteenth Edition, 2009. Prothioconazole provides control of diseases such as eyespot (*Pseudocercosporella herpotrichoides*), Fusarium ear blight (*Fusarium* spp., *Microdochium nivale*), leaf blotch diseases (*Zymoseptoria tritici, Parastagonospora nodorum, Pyrenophora* spp., *Rhynchosporium secalis,* etc.), rust (*Puccinia* spp.) and powdery mildew (*Blumeria graminis*), by foliar application, in wheat, barley and other crops.

As used herein, difenoconazole is the common name for 3-chloro-4-[(2*RS*, *4RS, 2RS,* 4*RS*)-4-methyl-2-(1*H*-1,2,4-triazol-1-ylmethyl)-1,3-dioxolan-2-yl]phenyl 4-chlorophenyl ether and possesses the following structure:

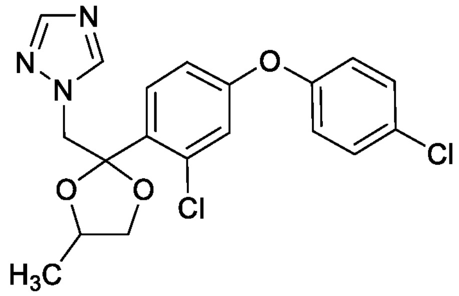

Its fungicidal activity is described in BCPC Online Pesticide Manual - Latest Version. Difenoconazole provides broad spectrum control, with preventive and curative action, of diseases caused by Ascomycetes, Basidiomycetes and Deuteromycetes in grapes, pome fruit, stone fruit, potatoes, sugar beet, oilseed rape, bananas, cereals, rice, soybeans, ornamentals and various vegetable crops.

As used herein, azoxystrobin is the common name for (*E*)-2-{2-[6-(2-cyanophenoxy)pyrimidin-4-yloxy]phenyl}-3-methoxyacrylate and possesses the following structure:

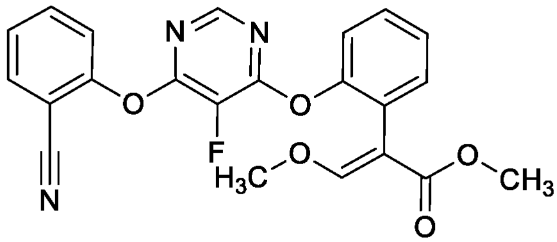

Its fungicidal activity is exemplified in The e-Pesticide Manual, Version 5.2, 2011. Exemplary uses of azoxystrobin include, but are not limited to, control of the following pathogens: *Erysiphe graminis, Puccinia* spp., *Parastagonospora nodorum, Zymoseptoria tritici* and *Pyrenophora teres* on temperate cereals; *Pyricularia oryzae* and *Rhizoctonia solani* on rice; *Plasmopara viticola* and *Uncinula necator* on vines; *Sphaerotheca fuliginea* and *Pseudoperonospora cubensis* on cucurbitaceae; *Phytophthora infestans* and *Alternaria solani* on potato and tomato; *Mycosphaerella arachidis, Rhizoctonia solani* and *Sclerotium rolfsii* on peanut; *Monilinia* spp. and *Cladosporium carpophilum* on peach; *Pythium* spp. and *Rhizoctonia solani* on turf; *Mycosphaerella* spp. on banana; *Cladosporium caryigenum* on pecan; *Elsinoë fawcettii, Colletotrichum* spp. and *Guignardia citricarpa* on citrus; *Colletotrichum* spp. and *Hemileia vastatrix* on coffee.

As used herein, pyraclostrobin is the common name for methyl *N*-[2-[[[1-(4-chlorophenyl)-1*H*-pyrazol-3-yl]oxy]methyl]phenyl]-*N*-methoxycarbamate and possesses the following structure:

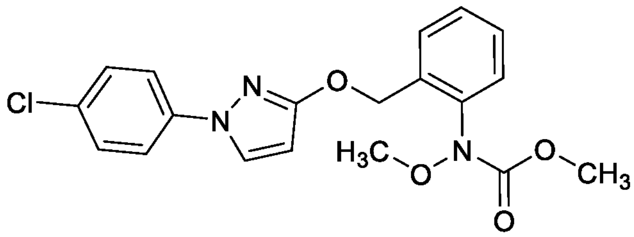

Its fungicidal activity is described in BCPC Online Pesticide Manual - Latest Version. Exemplary uses of pyraclostrobin include, but are not limited to, broad spectrum disease control of major plant pathogens, including *Zymoseptoria tritici, Puccinia* spp., *Drechslera tritici-repentis, Pyrenophora teres, Rhynchosporium secalis* and *Septoria nodorum* in cereals; *Mycosphaerella* spp. in peanuts; *Septoria glycines, Cercospora kikuchii* and *Phakopsora pachyrhizi* in soybeans; *Plasmopara viticola* and *Erysiphe necator* in grapes; *Phytophthora infestans* and *Alternaria solani* in potatoes and tomatoes; *Sphaerotheca fuliginea* and *Pseudoperonospora cubensis* in cucumber; *Mycosphaerella fijiensis* in bananas; *Elsinoë fawcettii* and *Guignardia citricarpa* in citrus and *Rhizoctonia solani* and *Pythium aphanidermatum* in turf.

As used herein, picoxystrobin is the common name for methyl (*E*)-3-methoxy-2-[2-(6-trifluoromethyl-2-pyridyloxymethyl)phenyl]acrylate and possesses the following structure:

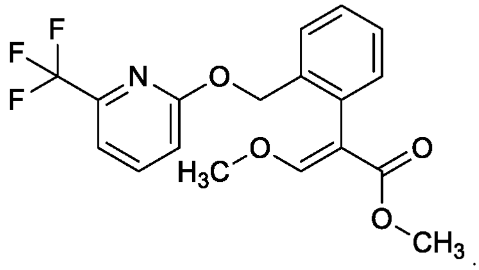

Its fungicidal activity is described in The e-Pesticide Manual, Version 5.2, 2011. Exemplary uses of picoxystrobin include, but are not limited to, broad-spectrum disease control in cereals, including *Mycosphaerella graminicola, Phaeosphaeria nodorum, Puccinia recondita* (brown rust), *Helminthosporium tritici-repentis* (tan spot) and *Blumeria graminis* f.sp. *tritici* (strobilurin-sensitive powdery mildew) in wheat; *Helminthosporium teres* (net blotch), *Rhynchosporium secalis, Puccinia hordei* (brown rust) and *Erysiphe graminis* f.sp. *hordei* (strobilurin-sensitive powdery mildew) in barley; *Puccinia coronata* and *Helminthosporium avenae* in oats; and *Puccinia recondita* and *Rhynchosporium secalis* in rye.

As used herein, fluxapyroxad is the common name for 3-(difluoromethyl)-1-methyl-*N*-(3',4',5'-trifluorobiphenyl-2-yl)pyrazole-4-carboxamide and possesses the following structure:

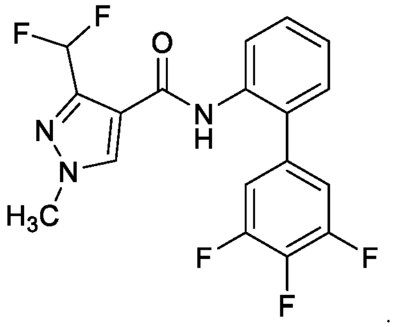

Its fungicidal activity is exemplified in Agrow Intelligence (https://www.agra-net.net/agra/agrow/databases/agrow-intelligence/). Exemplary uses of fluxapyroxad include, but are not limited to, the control of plant pathogens, such as *Helminthosporium teres* (net blotch), *Rhynchosporium secalis* (leaf scald), *Puccinia hordei* (brown rust), and *Erysiphe graminis* f.sp. *hordei* (powdery mildew) in a range of crops, such as barley, maize, and soybeans.

As used herein, benzovindiflupyr is the common name for *N*-[(1*RS,*4*SR*)-9-(dichloromethylene)-1,2,3,4-tetrahydro-1,4-methanonaphthalen-5-yl]-3-(difluoromethyl)-1-methylpyrazole-4-carboxamide and possesses the following structure:

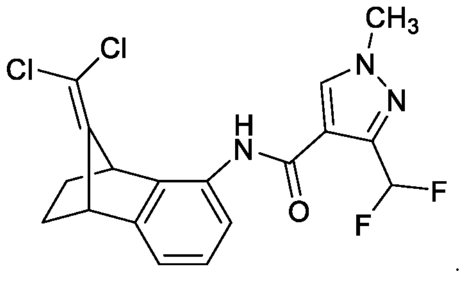

Its fungicidal activity is exemplified in Agrow Intelligence (https://www.agra-net.net/agra/agrow/databases/agrow-intelligence/). Exemplary uses of benzovindiflupyr include, but are not limited to, controlling a variety of pathogens such as *Botrytis* spp., *Erysiphe* spp., *Rhizoctonia* spp., *Septoria* spp., *Phytophthora* spp., *Pythium* spp., *Phakopsora pachyrhizi,* and *Puccinia recondita,* in a range of crops including vines, cereals, soybeans, cotton, and fruit and vegetable crops.

As used herein, penthiopyrad is the common name for *N*-[2-(1,3-dimethylbutyl)-3-thienyl]-1-methyl-3-(trifluoromethyl)-1*H*-pyrazole-4-carboxamide and possesses the following structure:

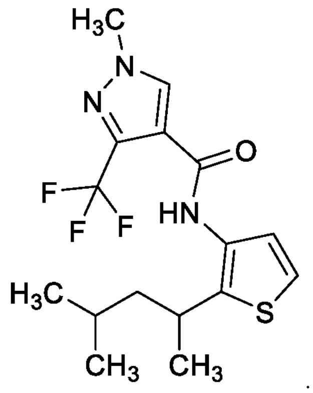

Its fungicidal activity is described in The Pesticide Manual, Fourteenth Edition, 2006. Penthiopyrad provides control of rust and Rhizoctonia diseases, as well as grey mold, powdery mildew and apple scab.

As used herein, bixafen is the common name for *N*-(3',4'-dichloro-5-fluoro[1,1'-biphenyl]-2-yl)-3-(difluoromethyl)-1-methyl-1*H*-pyrazole-4-carboxamide and possesses the following structure:

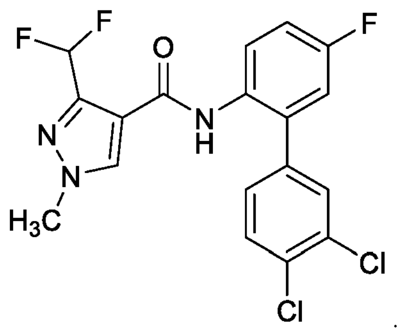

Its fungicidal activity is described in BCPC Online Pesticide Manual - Latest Version. Exemplary uses of bixafen include, but are not limited to, broad-spectrum disease control in cereals, including *Zymoseptoria tritici, Puccinia triticina, Puccinia striiformis, Oculimacula* spp. and *Pyrenophora tritici-repentis* in wheat and against *Pyrenophora teres, Ramularia collo-cygni, Rhynchosporium secalis* and *Puccinia hordei* in barley.

As used herein, chlorothalonil is the common name for tetrachloroisophthal-onitrile and possesses the following structure:

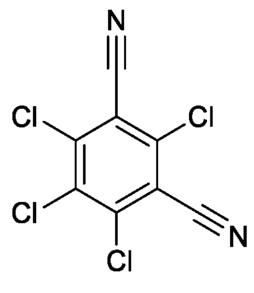

Its fungicidal activity is described in The Pesticide Manual, Fifteenth Edition, 2009. Chlorothalonil provides control of many fungal diseases in a wide range of crops, including pome fruit, stone fruit, almonds, citrus fruit, bush and cane fruit, cranberries, strawberries, pawpaws, bananas, mangoes, coconut palms, oil palms, rubber, pepper, vines, hops, vegetables, cucurbits, tobacco, coffee, tea, rice, soya beans, peanuts, potatoes, sugar beet, cotton, maize, ornamentals, mushrooms, and turf.

As used herein, mancozeb is the common name for [[2-[(dithiocarboxy)amino]ethyl]carbamodithioato(2-)-κ*S*,κ*S*']manganese mixture with [[2-[(dithiocarboxy)amino]ethyl]carbamodithioato(2-)-κ*S*,κ*S*']zinc and possesses the following structure:

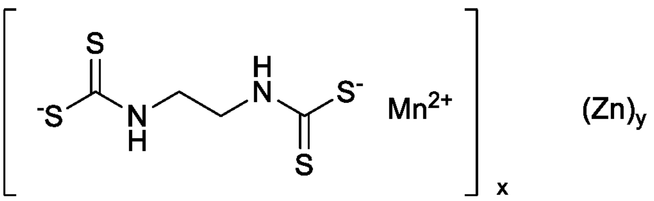

x:y = 1 : 0.091

Its fungicidal activity is described in The Pesticide Manual, Fifteenth Edition, 2009. Mancozeb provides control of a wide range of fungal pathogens on a variety of fruits, vegetables and field crops.

In the compositions described herein, the concentration ratio of the mixture of the compound of Formula I to other fungicides at which the fungicidal effect is synergistic against tomato early blight caused by *Alternaria solani* (ALTESO) in protectant applications lies within the range from about 50:1 to about 1:20.

In the compositions described herein, the concentration ratio of the mixture of the compound of Formula I to a sterol biosynthesis inhibitor at which the fungicidal effect is synergistic against ALTESO in protectant applications lies within the range from about 10:1 to about 1:1.6. In one embodiment, the concentration ratio of the mixture of the compound of Formula I to epoxiconazole at which the fungicidal effect is synergistic against ALTESO in protectant applications is from about 10:1 to about 1:1.6. In another embodiment, the concentration ratio of the mixture of the compound of Formula I to prothioconazole at which the fungicidal effect is synergistic against ALTESO in protectant applications is from about 10:1 to about 1.25:1. In yet another embodiment, the concentration ratio of the mixture of the compound of Formula I to difenoconazole at which the fungicidal effect is synergistic against ALTESO in protectant applications is from about 5:1 to about 1:1.6.

In the compositions described herein, the concentration ratio of the mixture of the compound of Formula I to a strobilurin fungicide at which the fungicidal effect is synergistic against ALTESO in protectant applications lies within the range from about 50:1 to about 1:1.6. In one embodiment, the concentration ratio of the mixture of the compound of Formula I to azoxystrobin at which the fungicidal effect is synergistic against ALTESO in protectant applications lies within the range from about 50:1 to about 3:1. In another embodiment, the concentration ratio of the mixture of the compound of Formula I to pyraclostrobin at which the fungicidal effect is synergistic against ALTESO in protectant applications lies within the range from about 50:1 to about 3:1. In yet another embodiment, the concentration ratio of the mixture of the compound of Formula I to picoxystrobin at which the fungicidal effect is synergistic against ALTESO in protectant applications lies within the range from about 10:1 to about 1:1.6.

In the compositions described herein, the concentration ratio of the mixture of the compound of Formula I to a succinate dehydrogenase inhibitor (SDHI) fungicide at which the fungicidal effect is synergistic against ALTESO in protectant applications lies within the range from about 50:1 to about 1:1.6. In one embodiment, the concentration ratio of the mixture of the compound of Formula I to fluxapyroxad at which the fungicidal effect is synergistic against ALTESO in protectant applications lies within the range from about 50:1 to about 3:1. In another embodiment, the concentration ratio of the mixture of the compound of Formula I to benzovindiflupyr at which the fungicidal effect is synergistic against ALTESO in protectant applications is from about 10:1 to about 1:1.6. In other embodiments, the concentration ratio of the mixture of the compound of Formula I to penthiopyrad at which the fungicidal effect is synergistic against ALTESO in protectant applications is from about 10:1 to about 1.3:1. In yet another embodiment, the concentration ratio of the mixture of the compound of Formula I to bixafen at which the fungicidal effect is synergistic against ALTESO in protectant applications is from about 10:1 to about 1:1.6.

In the compositions described herein, the concentration ratio of the mixture of the compound of Formula I to a multi-site inhibitor fungicide at which the fungicidal effect is synergistic against ALTESO in protectant applications lies within the range from about 1:1.3 to about 1:20. In one embodiment, the concentration ratio of the mixture of the compound of Formula I to chlorothalonil at which the fungicidal effect is synergistic against ALTESO in protectant applications lies within the range from about 1:1.3 to about 1:20. In another embodiment, the concentration ratio of the mixture of the compound of Formula I to mancozeb at which the fungicidal effect is synergistic against ALTESO in protectant applications lies within the range from about 1:1.3 to about 1:20.

The rate at which the synergistic composition is applied will depend upon the particular type of fungus to be controlled, the degree of control required and the timing and method of application. In general, the compositions described herein can be applied at an application rate of between about 40 grams per hectare (g/ha) and about 2650 g/ha based on the total amount of active ingredients in the composition.

The compositions comprising the compound of Formula I and a sterol biosynthesis inhibitor can be applied at an application rate of between about 40 g/ha and about 400 g/ha based on the total amount of active ingredients in the

composition. Epoxiconazole is applied at a rate of between about 50 g/ha and about 250 g/ha and the compound of Formula I is applied at a rate between about 10 g/ha and about 150 g/ha. Prothioconazole is applied at a rate of between about 50 g/ha and about 250 g/ha and the compound of Formula I is applied at a rate between about 10 g/ha and about 150 g/ha. Difenoconazole is applied at a rate of between about 30 g/ha and about 125 g/ha and the compound of Formula I is applied at a rate between about 10 g/ha and about 150 g/ha.

The compositions comprising the compound of Formula I and a strobilurin fungicide can be applied at an application rate of between about 40 g/ha and about 350 g/ha based on the total amount of active ingredients in the composition. Azoxystrobin is applied at a rate of between about 30 g/ha and about 200 g/ha and the compound of Formula I is applied at a rate between about 10 g/ha and about 150 g/ha. Pyraclostrobin is applied at a rate of between about 30 g/ha and about 200 g/ha and the compound of Formula I is applied at a rate between about 10 g/ha and about 150 g/ha. Picoxystrobin is applied at a rate of between about 30 g/ha and about 200 g/ha and the compound of Formula I is applied at a rate between about 10 g/ha and about 150 g/ha.

The compositions comprising the compound of Formula I and a carboxamide SDHI fungicide can be applied at an application rate of between about 40 g/ha and about 350 g/ha based on the total amount of active ingredients in the composition. Fluxapyroxad is applied at a rate of between about 45 g/ha and about 200 g/ha and the compound of Formula I is applied at a rate between about 10 g/ha and about 150 g/ha. Benzovindiflupyr is applied at a rate of between about 45 g/ha and about 200 g/ha and the compound of Formula I is applied at a rate between about 10 g/ha and about 150 g/ha. Penthiopyrad is applied at a rate of between about 45 g/ha and about 200 g/ha and the compound of Formula I is applied at a rate between about 10 g/ha and about 150 g/ha. Bixafen is applied at a rate of between about 30 g/ha and about 200 g/ha and the compound of Formula I is applied at a rate between about 10 g/ha and about 150 g/ha.

The compositions comprising the compound of Formula I and a multi-site inhibitor can be applied at an application rate of between about 1010 g/ha and about 2650 g/ha based on the total amount of active ingredients in the composition. Chlorothalonil is applied at a rate of between about 1000 g/ha and about 2500 g/ha and the compound of Formula I is applied at a rate between about 10 g/ha and about 150 g/ha. Mancozeb is applied at a rate of between about 1000 g/ha and about 2500 g/ha and the compound of Formula I is applied at a rate between about 10 g/ha and about 150 g/ha.

The components of the synergistic mixture described herein can be applied either separately or as part of a multipart fungicidal system.

The synergistic mixture of the present disclosure can be applied in conjunction with one or more other fungicides to control a wider variety of undesirable diseases. When used in conjunction with other fungicide(s), the compounds of the synergistic mixture of the present disclosure may be formulated with the other fungicide(s), tank mixed with the other fungicide(s) or applied sequentially with the other fungicide(s). Such other fungicides may include 2-(thiocyanatomethylthio)-benzothiazole, 2-phenylphenol, 8-hydroxyquinoline sulfate, ametoctradin, amisulbrom, antimycin, *Ampelomyces quisqualis,* azaconazole, *Bacillus subtilis, Bacillus subtilis* strain QST713, benalaxyl, benomyl, benthiavalicarb-isopropyl, benzylaminobenzene-sulfonate (BABS) salt, bicarbonates, biphenyl, bismerthiazol, bitertanol, blasticidin-S, borax, Bordeaux mixture, boscalid, bromuconazole, bupirimate, calcium polysulfide, captafol, captan, carbendazim, carboxin, carpropamid, carvone, chlazafenone, chloroneb, chlozolinate, *Coniothyrium minitans,* copper hydroxide, copper octanoate, copper oxychloride, copper sulfate, copper sulfate (tribasic), cuprous oxide, cyazofamid, cyflufenamid, cymoxanil, cyproconazole, cyprodinil, dazomet, debacarb, diammonium ethylenebis-(dithiocarbamate), dichlofluanid, dichlorophen, diclocymet, diclomezine, dichloran, diethofencarb, difenzoquat ion, diflumetorim, dimethomorph, dimoxystrobin, diniconazole, diniconazole-M, dinobuton, dinocap, diphenylamine, dipymetitrone, dithianon, dodemorph, dodemorph acetate, dodine, dodine free base, edifenphos, enestrobin, enestroburin, ethaboxam, ethoxyquin, etridiazole, famoxadone, fenamidone, fenarimol, fenbuconazole, fenfuram, fenhexamid, fenoxanil, fenpiclonil, fenpropidin, fenpropimorph, fenpyrazamine, fentin, fentin acetate, fentin hydroxide, ferbam, ferimzone, fluazinam, fludioxonil, fluindapyr, flumorph, fluopicolide, fluopyram, fluoroimide, fluoxastrobin, fluquinconazole, flusilazole, flusulfamide, flutianil, flutolanil, flutriafol, folpet, formaldehyde, fosetyl, fosetyl-aluminium, fuberidazole, furalaxyl, furametpyr, guazatine, guazatine acetates, GY-81, hexachlorobenzene, hexaconazole, hymexazol, imazalil, imazalil sulfate, imibenconazole, iminoctadine, iminoctadine triacetate, iminoctadine tris(albesilate), iodocarb, ipconazole, ipfenpyrazolone, iprobenfos, iprodione, iprovalicarb, isofetamide, isoprothiolane, isopyrazam, isotianil, kasugamycin, kasugamycin hydrochloride hydrate, kresoxium-methyl, laminarin, mancopper, mandipropamid, maneb, mefenoxam, mepanipyrim, mepronil, meptyl-dinocap, mercuric chloride, mercuric oxide, mercurous chloride, metalaxyl, metalaxyl-M, metam, metam-ammonium, metam-potassium, metam-sodium, metconazole, methasulfocarb, methyl iodide, methyl isothiocyanate, metiram, metominostrobin, metrafenone, mildiomycin, myclobutanil, nabam, nitrothal-isopropyl, nuarimol, octhilinone, ofurace, oleic acid (fatty acids), orysastrobin, oxadixyl, oxathiapiprolin, oxine-copper, oxpoconazole fumarate, oxycarboxin, pefurazoate, penconazole, pencycuron, penflufen, pentachlorophenol, pentachlorophenyl laurate, phenylmercury acetate, phosphonic acid, phthalide, polyoxin B, polyoxins, polyoxorim, potassium bicarbonate, potassium hydroxyquinoline sulfate, probenazole, prochloraz, procymidone, propamocarb, propamocarb hydrochloride, propiconazole, propineb, proquinazid, pydiflumetofen, pyrametostrobin, pyraoxystrobin, pyraziflumid, pyrazophos, pyribencarb, pyributicarb, pyrifenox, pyrimethanil, pyriofenone, pyroquilon, quinoclamine, quinoxyfen, quintozene, *Reynoutria sachalinensis* extract, sedaxane, silthiofam, simeconazole,

sodium 2-phenylphenoxide, sodium bicarbonate, sodium pentachlorophenoxide, spiroxamine, sulfur, SYP-Z048, tar oils, tebuconazole, tebufloquin, tecnazene, tetraconazole, thiabendazole, thifluzamide, thiophanate-methyl, thiram, tiadinil, tolclofos-methyl, tolylfluanid, triadimefon, triadimenol, triazoxide, tricyclazole, tridemorph, trifloxystrobin, triflumizole, triforine, triticonazole, validamycin, valifenalate, valiphenal, vinclozolin, zineb, ziram, zoxamide, *Candida oleophila, Fusarium oxysporum, Gliocladium* spp., *Phlebiopsis gigantea, Streptomyces griseoviridis, Trichoderma* spp., (*RS*)-*N*-(3,5-dichlorophenyl)-2-(methoxymethyl)-succinimide, 1,2-dichloropropane, 1,3-dichloro-1,1,3,3-tetrafluoroacetone hydrate, 1-chloro-2,4-dinitronaphthalene, 1-chloro-2-nitropropane, 2-(2-heptadecyl-2-imidazolin-1-yl)ethanol, 2,3-dihydro-5-phenyl-1,4-dithi-ine 1,1,4,4-tetraoxide, 2-methoxyethylmercury acetate, 2-methoxyethylmercury chloride, 2-methoxyethylmercury silicate, 3-(4-chlorophenyl)-5-methylrhodanine, 4-(2-nitroprop-1-enyl)phenyl thiocyanateme, aminopyrifen, ampropylfos, anilazine, azithiram, barium polysulfide, Bayer 32394, benodanil, benquinox, bentaluron, benzamacril; benzamacril-isobutyl, benzamorf, binapacryl, bis(methylmercury) sulfate, bis(tributyltin) oxide, buthiobate, cadmium calcium copper zinc chromate sulfate, carbamorph, CECA, chlobenthiazone, chloraniformethan, chlorfenazole, chlorquinox, climbazole, copper bis(3-phenylsalicylate), copper zinc chromate, cufraneb, cupric hydrazinium sulfate, cuprobam, cyclafuramid, cypendazole, cyprofuram, decafentin, dichlobentiazox, dichlone, dichlozoline, diclobutrazol, dimethirimol, dinocton, dinosulfon, dinoterbon, dipyrithione, ditalimfos, dodicin, drazoxolon, EBP, ESBP, etaconazole, etem, ethirim, fenaminosulf, fenapanil, fenitropan, fluindapyr, fluopimomide, fluotrimazole, furcarbanil, furconazole, furconazole-cis, furmecyclox, furophanate, glyodine, griseofulvin, halacrinate, Hercules 3944, hexylthiofos, ICIA0858, inpyrfluxam, ipfentrifluconazole, ipflufenoquin, isoflucypram, isopamphos, isovaledione, mandestrobin, mebenil, mecarbinzid, mefentrifluconazole, metazoxolon, methfuroxam, methylmercury dicyandiamide, metsulfovax, metyltetraprole, milneb, mucochloric anhydride, myclozolin, *N*-3,5-dichlorophenyl-succinimide, *N*-3-nitrophenylitaconimide, natamycin, *N*-ethylmercurio-4-toluenesulfonanilide, nickel bis(dimethyldithiocarbamate), OCH, phenylmercury dimethyldithiocarbamate, phenylmercury nitrate, phosdiphen, prothiocarb; prothiocarb hydrochloride, pydiflumetofen, pyracarbolid, pyrapropoyne, pyridachlometyl, pyridinitril, pyroxychlor, pyroxyfur, quinacetol; quinacetol sulfate, quinazamid, quinconazole, quinofumelin, rabenzazole, salicylanilide, SSF-109, sultropen, tecoram, thiadifluor, thicyofen, thiochlorfenphim, thiophanate, thioquinox, tioxymid, triamiphos, triarimol, triazbutil, trichlamide, urbacid, zarilamid, and any combinations thereof.

The compositions of the present disclosure are preferably applied in the form of a formulation comprising a composition of (a) a compound of Formula I and (b) at least one fungicide selected from the group consisting of epoxiconazole, prothioconazole, difenoconazole, azoxystrobin, pyraclostrobin, picoxystrobin, fluxapyroxad, benzovindiflupyr, picoxystrobin, bixafen, mancozeb, and chlorothalonil, together with a phytologically acceptable carrier.

Concentrated formulations can be dispersed in water, or another liquid, for application, or formulations can be dust-like or granular, which can then be applied without further treatment. The formulations are prepared according to procedures which are conventional in the agricultural chemical art, but which are novel and important because of the presence therein of a synergistic composition.

The formulations that are applied most often are aqueous suspensions or emulsions. Either such water-soluble, water-suspendable, or emulsifiable formulations are solids, usually known as wettable powders, or liquids, usually known as emulsifiable concentrates, aqueous suspensions, or suspension concentrates. The present disclosure contemplates all vehicles by which the synergistic compositions can be formulated for delivery and use as a fungicide.

As will be readily appreciated, any material to which these synergistic compositions can be added may be used, provided they yield the desired utility without significant interference with the activity of these synergistic compositions as antifungal agents.

Wettable powders, which may be compacted to form water-dispersible granules, comprise an intimate mixture of the synergistic composition, a carrier and agriculturally acceptable surfactants. The concentration of the synergistic composition in the wettable powder is usually from about 10% to about 90% by weight, more preferably about 25% to about 75% by weight, based on the total weight of the formulation. In the preparation of wettable powder formulations, the synergistic composition can be compounded with any of the finely divided solids, such as prophyllite, talc, chalk, gypsum, Fuller's earth, bentonite, attapulgite, starch, casein, gluten, montmorillonite clays, diatomaceous earths, purified silicates or the like. In such operations, the finely divided carrier is ground or mixed with the synergistic composition in a volatile organic solvent. Effective surfactants, comprising from about 0.5% to about 10% by weight of the wettable powder, include sulfonated lignins, naphthalenesulfonates, alkylbenzenesulfonates, alkyl sulfates, and non-ionic surfactants, such as ethylene oxide adducts of alkyl phenols.

Emulsifiable concentrates of the synergistic composition comprise a convenient concentration, such as from about 10% to about 50% by weight, in a suitable liquid, based on the total weight of the emulsifiable concentrate formulation. The components of the synergistic compositions, jointly or separately, are dissolved in a carrier, which is either a water-miscible solvent or a mixture of water-immiscible organic solvents, and emulsifiers. The concentrates may be diluted with water and oil to form spray mixtures in the form of oil-in-water emulsions. Useful organic solvents include aromatics, especially the high-boiling naphthalenic and olefinic portions of petroleum such as heavy aromatic naphtha. Other organic solvents may also be used, such as, for example, terpenic solvents, including rosin derivatives, aliphatic ketones, such as cyclohexanone, and complex alcohols, such as 2-ethoxyethanol.

Emulsifiers which can be advantageously employed herein can be readily determined by those skilled in the art and include various nonionic, anionic, cationic and amphoteric emulsifiers, or a blend of two or more emulsifiers. Examples of nonionic emulsifiers useful in preparing the emulsifiable concentrates include the polyalkylene glycol ethers and condensation products of alkyl and aryl phenols, aliphatic alcohols, aliphatic amines or fatty acids with ethylene oxide, propylene oxides such as the ethoxylated alkyl phenols and carboxylic esters solubilized with the polyol or polyoxyalkylene. Cationic emulsifiers include quaternary ammonium compounds and fatty amine salts. Anionic emulsifiers include the oil-soluble salts (e.g., calcium) of alkylaryl sulfonic acids, oil-soluble salts or sulfated polyglycol ethers and appropriate salts of phosphated polyglycol ether.

Representative organic liquids which can be employed in preparing the emulsifiable concentrates of the present disclosure are the aromatic liquids such as xylene, propyl benzene fractions, or mixed naphthalene fractions, mineral oils, substituted aromatic organic liquids such as dioctyl phthalate, kerosene, dialkyl amides of various fatty acids, particularly the dimethyl amides of fatty glycols and glycol derivatives such as the *n*-butyl ether, ethyl ether or methyl ether of diethylene glycol, and the methyl ether of triethylene glycol. Mixtures of two or more organic liquids are also often suitably employed in the preparation of the emulsifiable concentrate. The preferred organic liquids are xylene, and propyl benzene fractions, with xylene being most preferred. The surface-active dispersing agents are usually employed in liquid formulations and in the amount of from 0.1 to 20 percent by weight of the combined weight of the dispersing agent with the synergistic compositions. The formulations can also contain other compatible additives, for example, plant growth regulators and other biologically active compounds used in agriculture.

Aqueous suspensions comprise suspensions of one or more water-insoluble compounds, dispersed in an aqueous vehicle at a concentration in the range from about 5% to about 70% by weight, based on the total weight of the aqueous suspension formulation. Suspensions are prepared by finely grinding the components of the synergistic combination either together or separately, and vigorously mixing the ground material into a vehicle comprised of water and surfactants chosen from the same types discussed above. Other ingredients, such as inorganic salts and synthetic or natural gums, may also be added to increase the density and viscosity of the aqueous vehicle. It is often most effective to grind and mix at the same time by preparing the aqueous mixture and homogenizing it in an implement such as a sand mill, ball mill, or piston-type homogenizer.

The synergistic composition may also be applied as a granular formulation, which is particularly useful for applications to the soil. Granular formulations usually contain from about 0.5% to about 10% by weight of the compounds, based on the total weight of the granular formulation, dispersed in a carrier which consists entirely or in large part of coarsely divided attapulgite, bentonite, diatomite, clay or a similar inexpensive substance. Such formulations are usually prepared by dissolving the synergistic composition in a suitable solvent and applying it to a granular carrier which has been preformed to the appropriate particle size, in the range of from about 0.5 to about 3 millimeters (mm). Such formulations may also be prepared by making a dough or paste of the carrier and the synergistic composition, and crushing and drying to obtain the desired granular particle.

Dusts containing the synergistic composition are prepared simply by intimately mixing the synergistic composition in powdered form with a suitable dusty agricultural carrier, such as, for example, kaolin clay, ground volcanic rock, and the like. Dusts can suitably contain from about 1% to about 10% by weight of the synergistic composition/carrier combination.

The formulations may contain agriculturally acceptable adjuvant surfactants to enhance deposition, wetting and penetration of the synergistic composition onto the target crop and organism. These adjuvant surfactants may optionally be employed as a component of the formulation or as a tank mix. The amount of adjuvant surfactant will vary from 0.01 percent to 1.0 percent volume/volume (v/v) based on a spray-volume of water, preferably 0.05 to 0.5 percent. Suitable adjuvant surfactants include ethoxylated nonyl phenols, ethoxylated synthetic or natural alcohols, salts of the esters or sulfosuccinic acids, ethoxylated organosilicones, ethoxylated fatty amines and blends of surfactants with mineral or vegetable oils.

The formulations may optionally include combinations that can comprise at least 1% by weight of one or more of the synergistic compositions with another pesticidal compound. Such additional pesticidal compounds may be fungicides, insecticides, nematicides, miticides, arthropodicides, bactericides or combinations thereof that are compatible with the synergistic compositions of the present disclosure in the medium selected for application, and not antagonistic to the activity of the present compounds. Accordingly, in such embodiments the other pesticidal compound is employed as a supplemental toxicant for the same or for a different pesticidal use. The pesticidal compound and the synergistic composition can generally be mixed together in a weight ratio of from 1:100 to 100:1.

The present disclosure includes within its scope methods for the control or prevention of fungal attack. These methods comprise applying to the locus of the fungus, or to a locus in which the infestation is to be prevented (for example applying to tomato plants), a fungicidally effective amount of the synergistic composition. The synergistic composition is suitable for treatment of various plants at fungicidal levels, while exhibiting low phytotoxicity. The synergistic composition is useful in a protectant or eradicant fashion. The synergistic composition is applied by any of a variety of known techniques, either as the synergistic composition or as a formulation comprising the synergistic composition. For

example, the synergistic compositions may be applied to the roots, seeds or foliage of plants for the control of various fungi, without damaging the commercial value of the plants. The synergistic composition is applied in the form of any of the generally used formulation types, for example, as solutions, dusts, wettable powders, flowable concentrates, or emulsifiable concentrates. These materials are conveniently applied in various known fashions.

The synergistic composition has been found to have significant fungicidal effect for agricultural use.

In particular, the synergistic composition is effective in controlling a variety of undesirable fungi that infect useful plant crops. The synergistic composition may be used against a variety of *Ascomycete* and *Basidiomycete* fungi, including for example the following representative fungi species: leaf spot of sugar beets (*Cercospora beticola);* cucumber anthracnose (*Colletotrichum lagenarium*); cucumber powdery mildew (*Podosphaera xanthii*); watermelon stem gummy blight (*Didymella bryoniae*); grey mold (*Botrytis cinerea*); and Sclerotinia white mold (*Sclerotinia sclerotiorum*). It will be understood by those in the art that the efficacy of the synergistic compositions for one or more of the foregoing fungi establishes the general utility of the synergistic compositions as fungicides.

The synergistic compositions have a broad range of efficacy as a fungicide. The exact amount of the synergistic composition to be applied is dependent not only on the relative amounts of the components, but also on the particular action desired, the fungal species to be controlled, and the stage of growth thereof, as well as the part of the plant or other product to be contacted with the synergistic composition. Thus, formulations containing the synergistic composition may not be equally effective at similar concentrations or against the same fungal species.

The synergistic compositions are effective in use with plants in a disease-inhibiting and phytologically acceptable amount. The term "disease-inhibiting and phytologically acceptable amount" refers to an amount of the synergistic composition that kills or inhibits the plant disease for which control is desired, but is not significantly toxic to the plant. The exact concentration of synergistic composition required varies with the fungal disease to be controlled, the type of formulation employed, the method of application, the particular plant species, climate conditions, and the like.

The compositions can be applied to fungi or their locus by the use of conventional ground sprayers, granule applicators, and by other conventional means known to those skilled in the art.

The following examples are provided for illustrative purposes and should not be construed as limitations to the disclosure.

Examples

Evaluation of Protectant Activity of Fungicide Mixtures vs. Tomato Early Blight (*Alternaria solani*; Bayer code: ALTESO):

Tomato seedlings (variety Outdoor Girl) were propagated in a soil-less media (Metro mix™), with each pot having 1 plant, and used in the test when the first set of leaves was fully expanded. Treatments consisted of fungicide compounds epoxiconazole, prothioconazole, difenoconazole, azoxystrobin, pyraclostrobin, picoxystrobin, fluxapyroxad, benzovindiflupyr, penthiopyrad, bixafen, chlorothalonil, and mancozeb either using individually or as two-way mixture with the compound of Formula I.

The compounds were tested as technical grade material formulated in acetone, and spray solutions contained 10% acetone and 100 ppm Triton X-100. Fungicide solutions were applied onto plants using an automated booth sprayer, which utilized two 6218-1/4 JAUPM spray nozzles operating at 20 pounds per square inch (psi) set at opposing angles to cover both leaf surfaces. All sprayed plants were allowed to air dry prior to further handling. Control plants were sprayed in the same manner with the solvent blank.

Test plants were inoculated with an aqueous spore suspension of *Alternaria solani* 1 day after fungicide treatments (1-day protectant test). After inoculation the plants were kept in 100% relative humidity for two days to permit spores to germinate and infect the leaf. The plants were then transferred to a growth chamber for disease to develop. When the disease was fully developed on untreated plants, disease severity on the seedlings was assessed and activity was represented by percent of leaf area free of ALTESO infection relative to the untreated plants. The percent disease control was calculated using the equation (1-(disease severity on treated plants/disease severity on untreated plants))*100.

Colby's equation was used to determine the fungicidal effects expected from the mixtures. (See Colby, S. R. Calculation of the synergistic and antagonistic response of herbicide combinations. Weeds 1967, 15, 20-22.)

The following equation was used to calculate the expected activity of mixtures containing two active ingredients, A and B:

$$\text{Expected} = A + B - (A \times B/100)$$

A = observed efficacy of active component A at the same concentration as used in the mixture;
B = observed efficacy of active component B at the same concentration as used in the mixture.

Synergistic interactions between compound I and other fungicides were detected in protectant assays vs. ALTESO (Table 1).

Table 1: Synergistic Interactions of the Compound of Formula I and Other Fungicides in a 1-Day Protectant (1DP) *Alternaria solani* (ALTESO) Assay.

| Composition | Rates (ppm)* | ALTESO* | | Synergism Factor * |
|---|---|---|---|---|
| | | Observed* | Expected* | |
| Epoxiconazole + Compound I | 4+10 | 93.3 | 16.3 | 5.7 |
| Epoxiconazole + Compound I | 2 + 10 | 65.0 | 12.7 | 5.1 |
| Epoxiconazole + Compound I | 1 + 10 | 61.7 | 10.0 | 6.2 |
| Epoxiconazole + Compound I | 4+5 | 75.0 | 22.8 | 3.3 |
| Epoxiconazole + Compound I | 2+5 | 71.7 | 19.5 | 3.7 |
| Epoxiconazole + Compound I | 1 + 5 | 63.3 | 17.0 | 3.7 |
| Epoxiconazole + Compound I | 4 + 2.5 | 16.7 | 9.8 | 1.7 |
| Epoxiconazole + Compound I | 2 + 2.5 | 23.3 | 5.9 | 3.9 |
| Epoxiconazole + Compound I | 1 + 2.5 | 13.3 | 3.0 | 4.4 |
| Prothioconazole + Compound I | 2 + 10 | 33.3 | 10.0 | 3.3 |
| Prothioconazole + Compound I | 1 + 10 | 26.7 | 19.0 | 1.4 |
| Prothioconazole + Compound I | 4+5 | 60.0 | 41.9 | 1.4 |
| Prothioconazole + Compound I | 2+5 | 43.3 | 17.0 | 2.5 |
| Prothioconazole + Compound I | 1 + 5 | 56.7 | 25.3 | 2.2 |
| Prothioconazole + Compound I | 2 + 2.5 | 36.7 | 3.0 | 12.2 |
| Prothioconazole + Compound I | 1 + 2.5 | 46.7 | 12.7 | 3.7 |
| Difenoconazole + Compound I | 4+10 | 79.0 | 31.0 | 2.5 |
| Difenoconazole + Compound I | 2 + 10 | 50.0 | 34.0 | 1.5 |
| Difenoconazole + Compound I | 4+5 | 90.7 | 36.4 | 2.5 |
| Difenoconazole + Compound I | 2+5 | 63.3 | 39.1 | 1.6 |

(continued)

| Composition | Rates (ppm)* | ALTESO* | | Synergism Factor * |
|---|---|---|---|---|
| | | Observed* | Expected* | |
| Difenoconazole + Compound I | 4 + 2.5 | 53.3 | 25.6 | 2.1 |
| Difenoconazole + Compound I | 2 + 2.5 | 43.3 | 28.9 | 1.5 |
| Azoxystrobin + Compound I | 0.8 + 10 | 80.0 | 64.0 | 1.3 |
| Azoxystrobin + Compound I | 0.4 + 10 | 83.3 | 31.0 | 2.7 |
| Azoxystrobin + Compound I | 0.2 + 10 | 61.7 | 22.0 | 2.8 |
| Azoxystrobin + Compound I | 0.8 + 5 | 80.0 | 66.8 | 1.2 |
| Azoxystrobin + Compound I | 0.4 + 5 | 83.3 | 36.4 | 2.3 |
| Azoxystrobin + Compound I | 0.2 + 5 | 63.3 | 28.1 | 2.3 |
| Azoxystrobin + Compound I | 0.8 + 2.5 | 80.0 | 61.2 | 1.3 |
| Azoxystrobin + Compound I | 0.4 + 2.5 | 80.0 | 25.6 | 3.1 |
| Azoxystrobin + Compound I | 0.2 + 2.5 | 66.7 | 15.9 | 4.2 |
| Pyraclostrobin + Compound I | 0.8 + 10 | 78.3 | 31.0 | 2.5 |
| Pyraclostrobin + Compound I | 0.4 + 10 | 70.0 | 26.5 | 2.6 |
| Pyraclostrobin + Compound I | 0.2 + 10 | 66.7 | 13.0 | 5.1 |
| Pyraclostrobin + Compound I | 0.8 + 5 | 53.3 | 36.4 | 1.5 |
| Pyraclostrobin + Compound I | 0.4 + 5 | 60.0 | 32.2 | 1.9 |
| Pyraclostrobin + Compound I | 0.2 + 5 | 66.7 | 19.8 | 3.4 |
| Pyraclostrobin + Compound I | 0.8 + 2.5 | 86.7 | 25.6 | 3.4 |
| Pyraclostrobin + Compound I | 0.4 + 2.5 | 65.0 | 20.8 | 3.1 |
| Pyraclostrobin + Compound I | 0.2 + 2.5 | 43.3 | 6.2 | 7.0 |
| Picoxystrobin + Compound I | 4+10 | 97.3 | 55.0 | 1.8 |

(continued)

| Composition | Rates (ppm)* | ALTESO* | | Synergism Factor * |
|---|---|---|---|---|
| | | Observed* | Expected* | |
| Picoxystrobin + Compound I | 2 + 10 | 91.7 | 52.0 | 1.8 |
| Picoxystrobin + Compound I | 1 + 10 | 91.7 | 22.0 | 4.2 |
| Picoxystrobin + Compound I | 4+5 | 72.3 | 58.5 | 1.2 |
| Picoxystrobin + Compound I | 2+5 | 96.3 | 55.7 | 1.7 |
| Picoxystrobin + Compound I | 1 + 5 | 65.0 | 28.1 | 2.3 |
| Picoxystrobin + Compound I | 4 + 2.5 | 96.3 | 51.5 | 1.9 |
| Picoxystrobin + Compound I | 2 + 2.5 | 98.0 | 48.3 | 2.0 |
| Picoxystrobin + Compound I | 1 + 2.5 | 93.3 | 15.9 | 5.9 |
| Fluxapyroxad + Compound I | 0.8 + 10 | 46.7 | 19.0 | 2.5 |
| Fluxapyroxad + Compound I | 0.4 + 10 | 43.3 | 19.0 | 2.3 |
| Fluxapyroxad + Compound I | 0.2 + 10 | 53.3 | 31.0 | 1.7 |
| Fluxapyroxad + Compound I | 0.8 + 5 | 30.0 | 25.3 | 1.2 |
| Fluxapyroxad + Compound I | 0.8 + 2.5 | 33.3 | 12.7 | 2.6 |
| Fluxapyroxad + Compound I | 0.4 + 2.5 | 30.0 | 12.7 | 2.4 |
| Fluxapyroxad + Compound I | 0.2 + 2.5 | 33.3 | 25.6 | 1.3 |
| Benzovindiflupyr + Compound I | 4+10 | 58.3 | 23.5 | 2.5 |
| Benzovindiflupyr + Compound I | 2 + 10 | 53.3 | 13.0 | 4.1 |
| Benzovindiflupyr + Compound I | 1 + 10 | 30.0 | 16.0 | 1.9 |
| Benzovindiflupyr + Compound I | 4+5 | 50.0 | 29.5 | 1.7 |
| Benzovindiflupyr + Compound I | 2+5 | 56.7 | 19.8 | 2.9 |
| Benzovindiflupyr + Compound I | 1 + 5 | 53.3 | 22.5 | 2.4 |

(continued)

| Composition | Rates (ppm)* | ALTESO* | | Synergism Factor * |
|---|---|---|---|---|
| | | Observed* | Expected* | |
| Benzovindiflupyr + Compound I | 4 + 2.5 | 46.7 | 17.6 | 2.7 |
| Benzovindiflupyr + Compound I | 2 + 2.5 | 38.3 | 6.2 | 6.1 |
| Benzovindiflupyr + Compound I | 1 + 2.5 | 26.7 | 9.5 | 2.8 |
| Penthiopyrad + Compound I | 2 + 10 | 28.3 | 19.0 | 1.5 |
| Penthiopyrad + Compound I | 1 + 10 | 25.0 | 13.0 | 1.9 |
| Penthiopyrad + Compound I | 4+5 | 61.7 | 41.9 | 1.5 |
| Penthiopyrad + Compound I | 2+5 | 33.3 | 25.3 | 1.3 |
| Penthiopyrad + Compound I | 1 + 5 | 33.3 | 19.8 | 1.7 |
| Bixafen + Compound I | 4+10 | 81.7 | 61.0 | 1.3 |
| Bixafen + Compound I | 2 + 10 | 86.7 | 46.0 | 1.9 |
| Bixafen + Compound I | 1 + 10 | 73.3 | 49.0 | 1.5 |
| Bixafen + Compound I | 4+5 | 92.3 | 64.0 | 1.4 |
| Bixafen + Compound I | 2+5 | 78.3 | 50.2 | 1.6 |
| Bixafen + Compound I | 1 + 5 | 65.0 | 53.0 | 1.2 |
| Bixafen + Compound I | 4 + 2.5 | 96.7 | 58.0 | 1.7 |
| Bixafen + Compound I | 2 + 2.5 | 93.3 | 41.8 | 2.2 |
| Bixafen + Compound I | 1 + 2.5 | 94.0 | 45.0 | 2.1 |
| Chlorothalonil + Compound I | 50 + 10 | 56.7 | 25.0 | 2.3 |
| Chlorothalonil + Compound I | 25 + 10 | 53.3 | 22.0 | 2.4 |
| Chlorothalonil + Compound I | 12.5 + 10 | 55.0 | 13.0 | 4.2 |
| Chlorothalonil + Compound I | 50 + 2.5 | 26.7 | 19.2 | 1.4 |
| Chlorothalonil + Compound I | 12.5 + 2.5 | 13.3 | 6.2 | 2.1 |
| Mancozeb + Compound I | 50 + 10 | 66.7 | 17.5 | 3.8 |
| Mancozeb + Compound I | 25 + 10 | 56.7 | 11.5 | 4.9 |
| Mancozeb + Compound I | 12.5 + 10 | 55.0 | 10.0 | 5.5 |

(continued)

| Composition | Rates (ppm)* | ALTESO* | | Synergism Factor * |
|---|---|---|---|---|
| | | Observed* | Expected* | |
| Mancozeb + Compound I | 50 + 5 | 58.3 | 23.9 | 2.4 |
| Mancozeb + Compound I | 25 + 5 | 51.7 | 18.4 | 2.8 |
| Mancozeb + Compound I | 12.5 + 5 | 51.7 | 17.0 | 3.0 |
| Mancozeb + Compound I | 50 + 2.5 | 51.7 | 11.1 | 4.7 |
| Mancozeb + Compound I | 25 + 2.5 | 38.3 | 4.6 | 8.3 |
| Mancozeb + Compound I | 12.5 + 2.5 | 33.3 | 3.0 | 11.1 |

*ALTESO = Tomato early blight; *Alternaria solani*
*Observed = Observed percent disease control at the test rates
*Expected = Percent disease control expected as predicted by the Colby equation
*ppm = Parts per million
*Synergism factor = Observed / Expected

**Claims**

**1.** Use of a synergistic fungicidal mixture, comprising:
a fungicidally effective amount of the compound of Formula I, (S)-1,1-bis(4-fluorophenyl)propan-2-yl (3-acetoxy-4-methoxypicolinoyl)-L-alaninate:

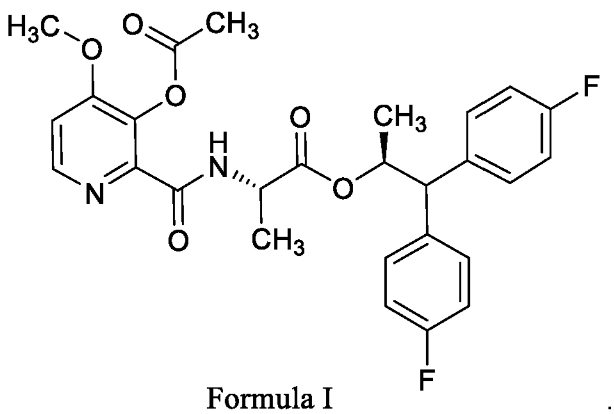

Formula I ;

and

at least one additional fungicide selected from the group consisting of sterol biosynthesis inhibitors, respiration inhibitors, and multi-site action inhibitors,
wherein the sterol biosynthesis inhibitor is selected from the group consisting of epoxiconazole, prothioconazole and difenoconazole; the respiration inhibitor is selected from the group consisting of azoxystrobin, pyraclostrobin and picoxystrobin or from the group consisting of fluxapyroxad, benzovindiflupyr, penthiopyrad and bixafen; or the multi-site inhibitor is selected from the group consisting of chlorothalonil and mancozeb,
for fungal control in vegetables.

**2.** The use of claim 1 wherein a concentration ratio of the compound of Formula I to epoxiconazole is from about 10:1 to about 1:1.6; or wherein a concentration ratio of the compound of Formula I to prothioconazole is from about 10:1 to about 1.3:1; or wherein a concentration ratio of the compound of Formula I to difenoconazole is from about 5:1 to about 1:1.6.

**3.** The use of claim 1 wherein a concentration ratio of the compound of Formula I to azoxystrobin is from about 50:1 to about 3.1:1; or wherein a concentration ratio of the compound of Formula I to pyraclostrobin is from about 50:1 to about 3.1:1, or wherein a concentration ratio of the compound of Formula I to picoxystrobin is from about 10:1 to about 1:1.6.

**4.** The use of claim 1 wherein a concentration ratio of the compound of Formula I to fluxapyroxad is from about 50:1 to about 3.1:1; or wherein a concentration ratio of the compound of Formula I to benzovindiflupyr is from about 10:1 to about 1:1.6; or wherein a concentration ratio of the compound of Formula I to penthiopyrad is from about 10:1 to about 1.3:1; or wherein a concentration ratio of the compound of Formula I to bixafen is from about 10:1 to about 1:1.6.

**5.** The use of claim 1 wherein a concentration ratio of the compound of Formula I to chlorothalonil is from about 1:1.3 to about 1:20; or wherein a concentration ratio of the compound of Formula I to mancozeb is from about 1:1.3 to about 1:20.

**6.** The use of any of claims 1 - 5 for controlling the fungal pathogen which is the causal agent of early blight of tomato *(Alternaria solani).*

**7.** A method for the control or prevention of fungal attack in vegetables comprising applying to the locus of the fungus or to a locus in which the infestation is to be prevented, a fungicidally effective amount of a synergistic, fungicidal composition, the composition comprising of a fungicidally effective amount of a mixture comprising a fungicidally effective amount of the compound of Formula I, (*S*)-1,1-bis(4-fluorophenyl)propan-2-yl (3-acetoxy-4-methoxypicolinoyl)-L-alaninate:

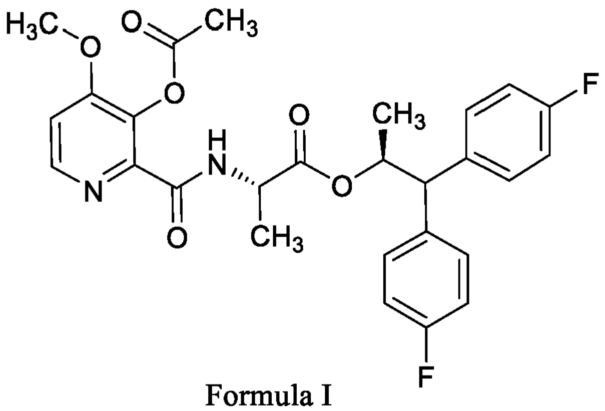

Formula I ;

and at least one additional fungicide selected from the group consisting of sterol biosynthesis inhibitors, respiration inhibitors, and multi-site action inhibitors, wherein the sterol biosynthesis inhibitor is selected from the group consisting of epoxiconazole, prothioconazole and difenoconazole; the respiration inhibitor is selected from the group consisting of azoxystrobin, pyraclostrobin and picoxystrobin or from the group consisting of fluxapyroxad, benzovindiflupyr, penthiopyrad and bixafen; or the multi-site inhibitor is selected from the group consisting of chlorothalonil and mancozeb; and an agriculturally acceptable carrier.

**8.** The method of claim 7 wherein a concentration ratio of the compound of Formula I to epoxiconazole is from about 10:1 to about 1:1.6; or wherein a concentration ratio of the compound of Formula I to prothioconazole is from about 10:1 to about 1.3:1; or wherein a concentration ratio of the compound of Formula I to difenoconazole is from about 5:1 to about 1:1.6.

**9.** The method of claim 7 wherein a concentration ratio of the compound of Formula I to azoxystrobin is from about 50:1 to about 3.1:1; or wherein a concentration ratio of the compound of Formula I to pyraclostrobin is from about 50:1 to about 3.1:1, or wherein a concentration ratio of the compound of Formula I to picoxystrobin is from about

10:1 to about 1:1.6.

**10.** The method of claim 7 wherein a concentration ratio of the compound of Formula I to fluxapyroxad is from about 50:1 to about 3.1:1; or wherein a concentration ratio of the compound of Formula I to benzovindiflupyr is from about 10:1 to about 1:1.6; or wherein a concentration ratio of the compound of Formula I to penthiopyrad is from about 10:1 to about 1.3:1; or wherein a concentration ratio of the compound of Formula I to bixafen is from about 10:1 to about 1:1.6.

**11.** The method of claim 7 wherein a concentration ratio of the compound of Formula I to chlorothalonil is from about 1:1.3 to about 1:20; or wherein a concentration ratio of the compound of Formula I to mancozeb is from about 1:1.3 to about 1:20.

**12.** The method of any of claims 7 - 11 wherein the fungal pathogen is the causal agent of early blight of tomato *(Alternaria solani).*

**Patentansprüche**

**1.** Verwendung einer synergistischen fungiziden Mischung umfassend:
eine fungizid wirksame Menge der Verbindung der Formel I, (S)-1,1-Bis(4-fluorphenyl)propan-2-yl(3-acetoxy-4-methoxypicolinoyl)-L-alaninat:

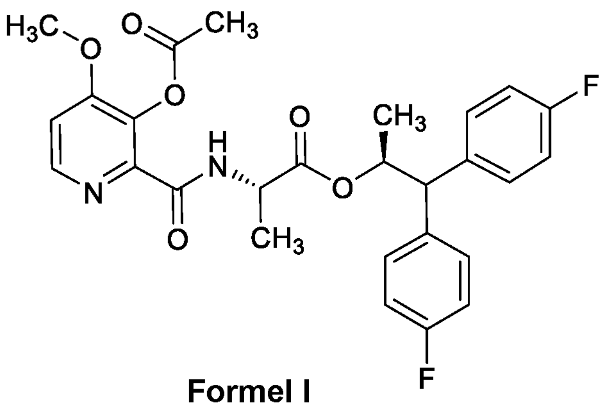

**Formel I**

;

und

wenigstens ein zusätzliches Fungizid, ausgewählt aus der Gruppe bestehend aus Hemmern der Sterolbiosynthese, Hemmern der Atmungskette und sog. Multi-Site-Hemmern,
wobei der Hemmer der Sterolbiosynthese aus der Gruppe bestehend aus Epoxiconazol, Prothioconazol und Difenoconazol ausgewählt ist, der Hemmer der Atmungskette aus der Gruppe bestehend aus Azoxystrobin, Pyraclostrobin und Picoxystrobin oder aus der Gruppe bestehend aus Fluxapyroxad, Benzovindiflupyr, Penthiopyrad und Bixafen ausgewählt ist;
oder der sog. Multi-Site-Hemmer aus der Gruppe bestehend aus Chlorthalonil und Mancozeb ausgewählt ist, zur Pilzbekämpfung in Gemüse.

**2.** Die Verwendung gemäß Anspruch 1, wobei ein Konzentrationsverhältnis der Verbindung der Formel I zu Epoxiconazol von etwa 10:1 bis etwa 1:1,6 beträgt; oder wobei ein Konzentrationsverhältnis der Verbindung der Formel I zu Prothioconazol von etwa 10:1 bis etwa 1,3:1 beträgt; oder wobei ein Konzentrationsverhältnis der Verbindung der Formel I zu Difenoconazol von etwa 5:1 bis etwa 1:1,6 beträgt.

**3.** Die Verwendung gemäß Anspruch 1, wobei ein Konzentrationsverhältnis der Verbindung der Formel I zu Azoxystrobin von etwa 50:1 bis etwa 3,1:1 beträgt; oder wobei ein Konzentrationsverhältnis der Verbindung der Formel I zu Pyraclostrobin von etwa 50:1 bis etwa 3,1:1 beträgt, oder wobei ein Konzentrationsverhältnis der Verbindung der Formel I zu Picoxystrobin von etwa 10:1 bis etwa 1:1,6 beträgt.

4. Die Verwendung gemäß Anspruch 1, wobei ein Konzentrationsverhältnis der Verbindung der Formel I zu Fluxapyroxad von etwa 50:1 bis etwa 3,1:1 beträgt; oder wobei ein Konzentrationsverhältnis der Verbindung der Formel I zu Benzovindiflupyr von etwa 10:1 bis etwa 1:1,6 beträgt; oder wobei ein Konzentrationsverhältnis der Verbindung der Formel I zu Penthiopyrad von etwa 10:1 bis etwa 1,3:1 beträgt; oder wobei ein Konzentrationsverhältnis der Verbindung der Formel I zu Bixafen von etwa 10:1 bis etwa 1:1,6 beträgt.

5. Die Verwendung gemäß Anspruch 1, wobei ein Konzentrationsverhältnis der Verbindung der Formel I zu Chlorthalonil von etwa 1:1,3 bis etwa 1:20 beträgt; oder wobei ein Konzentrationsverhältnis der Verbindung der Formel I zu Mancozeb von etwa 1:1,3 bis etwa 1:20 beträgt.

6. Die Verwendung gemäß irgendeinem der Ansprüche 1 bis 5 zur Bekämpfung des Pilzerregers, der die Dürrfleckenkrankheit der Tomate verursacht *(Alternaria solani).*

7. Ein Verfahren zur Bekämpfung oder Verhinderung eines Pilzbefalls von Gemüse, umfassend das Anwenden einer fungizid wirksamen Menge einer synergistischen fungiziden Zusammensetzung auf den Ort des Pilzes oder auf einen Ort, auf dem ein Befall verhindert werden soll, wobei die Zusammensetzung eine fungizid wirksame Menge einer Mischung umfassend eine fungizid wirksame Menge der Verbindung der Formel I, (*S*)-1,1-Bis(4-fluorphenyl)propan-2-yl(3-acetoxy-4-methoxypicolinoyl)-L-alaninat:

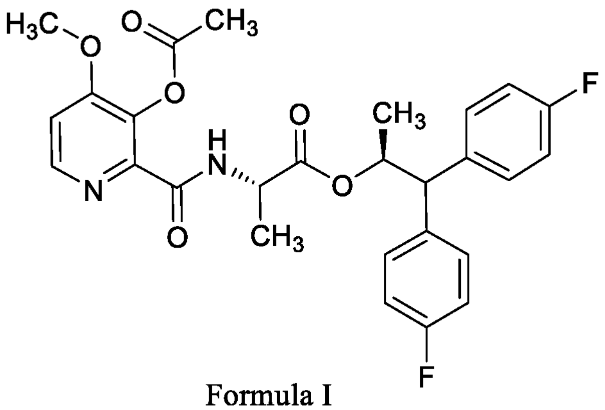

Formula I

;

und

wenigstens ein zusätzliches Fungizid, ausgewählt aus der Gruppe bestehend aus Hemmern der Sterolbiosynthese, Hemmern der Atmungskette und sog. Multi-Site-Hemmern,
wobei der Hemmer der Sterolbiosynthese aus der Gruppe bestehend aus Epoxiconazol, Prothioconazol und Difenoconazol ausgewählt ist, der Hemmer der Atmungskette aus der Gruppe bestehend aus Azoxystrobin, Pyraclostrobin und Picoxystrobin oder aus der Gruppe bestehend aus Fluxapyroxad, Benzovindiflupyr, Penthiopyrad und Bixafen ausgewählt ist; oder der sog. Multi-Site-Hemmer aus der Gruppe bestehend aus Chlorthalonil und Mancozeb ausgewählt ist, und einen landwirtschaftlich akzeptablen Trägerstoff.

8. Das Verfahren gemäß Anspruch 7, wobei ein Konzentrationsverhältnis der Verbindung der Formel I zu Epoxiconazol von etwa 10:1 bis etwa 1:1,6 beträgt; oder wobei ein Konzentrationsverhältnis der Verbindung der Formel I zu Prothioconazol von etwa 10:1 bis etwa 1,3:1 beträgt; oder wobei ein Konzentrationsverhältnis der Verbindung der Formel I zu Difenoconazol von etwa 5:1 bis etwa 1:1,6 beträgt.

9. Das Verfahren gemäß Anspruch 7, wobei ein Konzentrationsverhältnis der Verbindung der Formel I zu Azoxystrobin von etwa 50:1 bis etwa 3,1:1 beträgt; oder wobei ein Konzentrationsverhältnis der Verbindung der Formel I zu Pyraclostrobin von etwa 50:1 bis etwa 3,1:1 beträgt; oder wobei ein Konzentrationsverhältnis der Verbindung der Formel I zu Picoxystrobin von etwa 10:1 bis etwa 1:1,6 beträgt.

10. Das Verfahren gemäß Anspruch 7, wobei ein Konzentrationsverhältnis der Verbindung der Formel I zu Fluxapyroxad von etwa 50:1 bis etwa 3,1:1 beträgt, oder wobei ein Konzentrationsverhältnis der Verbindung der Formel I zu Benzovindiflupyr von etwa 10:1 bis etwa 1:1,6 beträgt; oder wobei ein Konzentrationsverhältnis der Verbindung der Formel I zu Penthiopyrad von etwa 10:1 bis etwa 1,3:1 beträgt; oder wobei ein Konzentrationsverhältnis der Verbindung der Formel I zu Bixafen von etwa 10:1 bis etwa 1:1,6 beträgt.

**11.** Das Verfahren gemäß Anspruch 7, wobei ein Konzentrationsverhältnis der Verbindung der Formel I zu Chlorthalonil von etwa 1:1,3 bis etwa 1:20 beträgt; oder wobei ein Konzentrationsverhältnis der Verbindung der Formel I zu Mancozeb von etwa 1:1,3 bis etwa 1:20 beträgt.

**12.** Das Verfahren gemäß irgendeinem der Ansprüche 7 bis 11, wobei der Pilzerreger der Erreger ist, der die Dürrfleckenkrankheit der Tomate verursacht *(Alternaria solani).*

## Revendications

**1.** Utilisation d'un mélange fongicide synergique, comprenant :
une quantité à effet fongicide du composé de formule I, (3-acétoxy-4-méthoxy-picolinoyl)-*L*-alaninate de (*S*)-1,1-bis(4-fluorophényl)propan-2-yle :

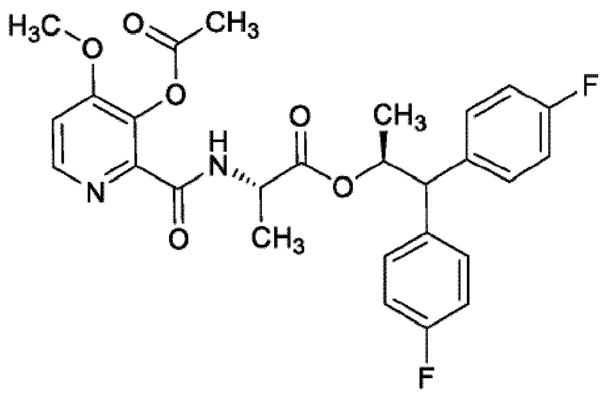

Formule I

; et

au moins un fongicide supplémentaire choisi dans l'ensemble constitué par les inhibiteurs de la biosynthèse des stérols, les inhibiteurs de la respiration, et les inhibiteurs à action multisite, l'inhibiteur de la biosynthèse des stérols étant choisi dans l'ensemble constitué par l'époxiconazole, le prothioconazole et le difénoconazole ; l'inhibiteur de la respiration étant choisi dans l'ensemble constitué par l'azoxystrobine, la pyraclostrobine et la picoxystrobine ou dans l'ensemble constitué par le fluxapyroxade, le benzovindiflupyr, le penthiopyrade et le bixafène ; ou l'inhibiteur multisite étant choisi dans l'ensemble constitué par le chlorothalonil et le mancozèbe, pour la lutte antifongique chez les légumes.

**2.** Utilisation selon la revendication 1, dans laquelle un rapport de concentrations du composé de formule I à l'époxiconazole vaut d'environ 10:1 à environ 1:1,6 ; ou dans laquelle un rapport de concentrations du composé de formule I au prothioconazole vaut d'environ 10:1 à environ 1,3:1 ; ou dans laquelle un rapport de concentrations du composé de formule I au difénoconazole vaut d'environ 5:1 à environ 1:1,6.

**3.** Utilisation selon la revendication 1, dans laquelle un rapport de concentrations du composé de formule I à l'azoxystrobine vaut d'environ 50:1 à environ 3,1:1 ; ou dans laquelle un rapport de concentrations du composé de formule I à la pyraclostrobine vaut d'environ 50:1 à environ 3,1:1 ; ou dans laquelle un rapport de concentrations du composé de formule I à la picoxystrobine vaut d'environ 10:1 à environ 1:1,6.

**4.** Utilisation selon la revendication 1, dans laquelle un rapport de concentrations du composé de formule I au fluxapyroxade vaut d'environ 50:1 à environ 3,1:1 ; ou dans laquelle un rapport de concentrations du composé de formule I au benzovindiflupyr vaut d'environ 10:1 à environ 1:1,6 ; ou dans laquelle un rapport de concentrations du composé de formule I au penthiopyrade vaut d'environ 10:1 à environ 1,3:1 ; ou dans laquelle un rapport de concentrations du composé de formule I au bixafène vaut d'environ 10:1 à environ 1:1,6.

**5.** Utilisation selon la revendication 1, dans laquelle un rapport de concentrations du composé de formule I au chlorothalonile vaut d'environ 1:1,3 à environ 1:20 ; ou dans laquelle un rapport de concentrations du composé de formule I au mancozèbe vaut d'environ 1:1,3 à environ 1:20.

6. Utilisation selon l'une quelconque des revendications 1 - 5 pour lutter contre l'agent pathogène fongique qui est l'agent causal de l'alternariose de la tomate (*Alternaria solani*).

7. Procédé pour la lutte contre ou la prévention de l'attaque fongique chez des légumes, comprenant le fait d'appliquer sur le lieu du champignon ou sur un lieu dans lequel doit être prévenue l'infestation, une quantité à effet fongicide d'une composition fongicide synergique, la composition comprenant une quantité à effet fongicide d'un mélange comprenant une quantité à effet fongicide du composé de formule I, (3-acétoxy-4-méthoxypicolinoyl)-L-alaninate de (*S*)-1,1-bis(4-fluoro-phényl)propan-2-yle :

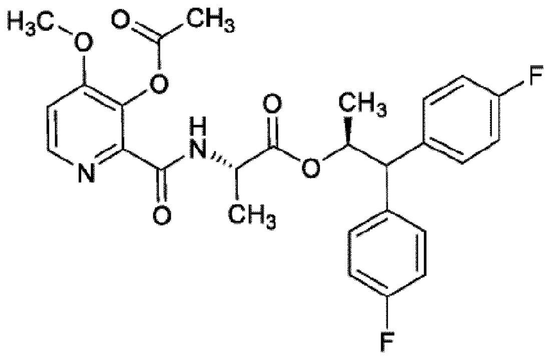

Formule I

; et au moins un fongicide supplémentaire choisi dans l'ensemble constitué par les inhibiteurs de la biosynthèse des stérols, les inhibiteurs de la respiration, et les inhibiteurs à action multisite, l'inhibiteur de la biosynthèse des stérols étant choisi dans l'ensemble constitué par l'époxiconazole, le prothioconazole et le difénoconazole ; l'inhibiteur de la respiration étant choisi dans l'ensemble constitué par l'azoxystrobine, la pyraclostrobine et la picoxystrobine ou dans l'ensemble constitué par le fluxapyroxade, le benzovindiflupyr, le penthiopyrade et le bixafène ; ou l'inhibiteur multisite étant choisi dans l'ensemble constitué par le chlorothalonil et le mancozèbe ; et une substance de support acceptable en agriculture.

8. Procédé selon la revendication 7, dans lequel un rapport de concentrations du composé de formule I à l'époxiconazole vaut d'environ 10:1 à environ 1:1,6 ; ou dans lequel un rapport de concentrations du composé de formule I au prothioconazole vaut d'environ 10:1 à environ 1,3:1 ; ou dans lequel un rapport de concentrations du composé de formule I au difénoconazole vaut d'environ 5:1 à environ 1:1,6.

9. Procédé selon la revendication 7, dans lequel un rapport de concentrations du composé de formule I à l'azoxystrobine vaut d'environ 50:1 à environ 3,1:1 ; ou dans lequel un rapport de concentrations du composé de formule I à la pyraclostrobine vaut d'environ 50:1 à environ 3,1:1 ; ou dans lequel un rapport de concentrations du composé de formule I à la picoxystrobine vaut d'environ 10:1 à environ 1:1,6.

10. Procédé selon la revendication 7, dans lequel un rapport de concentrations du composé de formule I au fluxapyroxade vaut d'environ 50:1 à environ 3,1:1 ; ou dans lequel un rapport de concentrations du composé de formule I au benzovindiflupyr vaut d'environ 10:1 à environ 1:1,6 ; ou dans lequel un rapport de concentrations du composé de formule I au penthiopyrade vaut d'environ 10:1 à environ 1,3:1 ; ou dans lequel un rapport de concentrations du composé de formule I au bixafène vaut d'environ 10:1 à environ 1:1,6.

11. Procédé selon la revendication 7, dans lequel un rapport de concentrations du composé de formule I au chlorothalonile vaut d'environ 1:1,3 à environ 1:20 ; ou dans lequel un rapport de concentrations du composé de formule I au mancozèbe vaut d'environ 1:1,3 à environ 1:20.

12. Procédé selon l'une quelconque des revendications 7 - 11, dans lequel l'agent pathogène fongique est l'agent causal de l'alternariose de la tomate (*Alternaria solani)*.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62500186


### Non-patent literature cited in the description


- The Pesticide Manual. 2009
- The e-Pesticide Manual. 2011
- The Pesticide Manual. 2006
- **COLBY, S. R.** Calculation of the synergistic and antagonistic response of herbicide combinations. *Weeds,* 1967, vol. 15, 20-22